# EUROPEAN PATENT APPLICATION

(11) **EP 1 864 689 A1**
(43) Date of publication of application: **12.12.2007**
(21) Application number: 06730558.1
(22) Date of filing: 23.03.2006
(51) Int. Cl.: A61M 25/01, C25D 13/06, C25D 13/12

(54) **MEDICAL INSTRUMENT AND METHOD OF PRODUCING THE SAME**

(30) Priority: 30.03.2005 JP 2005099006
(71) Applicant: Japan Lifeline Co., Ltd, Toshima-ku, Tokyo 171-0014 (JP)
(72) Inventor: HANAWA, Takao, Tokyo 1640011 (JP); KAWASAKI, Hironori c/o Japan Lifeline Co., Ltd., Tokyo 115-0051 (JP); KAWABATA, Takashi c/o Japan Lifeline Co., Ltd., Tokyo 115-0051 (JP)
(74) Representative: Prop, Gerrit
(86) International application number: PCT/JP2006/306610
(87) International publication number: WO 2006/106796

(57) **Abstract**

A medical appliance having a metal surface in which a hydrophilic organic compound having a polar group is directly fixed to the metal surface in accordance with an electrochemical reaction without intermediate layers; and a process for producing a medical appliance which comprises dipping a material for a medical appliance having a metal surface and an electrode into a solution comprising a hydrophilic organic compound having a polar group, and fixing the hydrophilic organic compound to the metal surface of the material for a medical appliance in accordance with an electrochemical reaction by applying a voltage between a cathode and an anode using the material for a medical appliance as the cathode and the electrode as the anode. The medical appliance exhibits excellent lubricating property since the metal surface is tightly coated with a thin layer of the hydrophilic organic compound. Therefore, excellent operability is exhibited in insertion into blood vessels, and the possibility of causing damages on inner layers of blood vessels is small. The medical appliance can be produced in simple steps in accordance with the process.

## Description

### TECHNICAL FIELD

The present invention relates to a medical appliance and a process for producing the appliance. More particularly, the present invention relates to a medical appliance having a metal surface which exhibits excellent lubricating property by tightly coating the metal surface with a thin layer of the hydrophilic organic compound, provides excellent operability in insertion into blood vessels and has small possibility of causing damages on inner layers of blood vessels and a process for producing the medical appliance which can produce the appliance in simple steps.

### BACKGROUND ART

In medical treatments such as the percutaneous transluminal coronary angioplasty (PTCA) and the stent angioplasty and examinations such as the cardiac angiography, a catheter is inserted into a prescribed position in a blood vessel. Although a catheter is formed with a flexible material, the blood vessel into which the catheter is inserted has a complicatedly curved shape, and it is difficult that the catheter is pushed into the prescribed position in the blood vessel when the catheter is used alone. Therefore, it is widely conducted that a guidewire is inserted into the blood vessel, and the catheter is pushed into the prescribed position in the blood vessel along the guidewire.

To the guidewire, various properties such as the property for insertion which requires a small resistance to pushing-in so that the guidewire can be moved forward easily without causing damages on the inner layer of the blood vessel, the property for transmitting torque which requires easy transmission of the torque of rotation at the handling portion to the tip portion so that the guidewire can be moved into the target blood vessel by suitably selecting a branch of the blood vessel, the small permanent set which prevents formation of permanent set by deformation when the guidewire passes through a curved portion of the blood vessel so that the target blood vessel can be easily selected at a branch of the blood vessel beyond the curved portion, and the property for preventing trapping which requires a small friction coefficient at the tip portion and a smooth surface so that the possibility of being caught with stent strata is small, are required.

To provide the lubricating property to the surface of a guidewire and improve the property for insertion of the guidewire, it has been conducted that the surface of the guidewire is coated with a hydrophilic macromolecule such as polyethylene glycol and polyvinylpyrrolidone. However, the affinity between the guidewire made of a metal and the hydrophilic macromolecule is small, and it is difficult that the surface of the guidewire is directly coated with the hydrophilic macromolecule. Therefore, it is widely conducted that an intermediate layer is formed on the surface of the guidewire, and the formed intermediate layer is coated with the hydrophilic macromolecule. For example, as the process for producing a guidewire which allows easy operation of insertion into a tubular organ of a human body by decreasing the friction coefficient, a process in which the outer periphery of a core wire is coated with a synthetic resin reactive to isocyanate group, unreacted isocyanate group is introduced to the surface of the coating film of the synthetic resin by bonding a compound having isocyanate group to the surface of the coating film of the synthetic resin, and polyethylene glycol is bonded to the surface of the coating film of the synthetic resin via the isocyanate group to form a hydrophilic coating film, is proposed (Patent Reference 1). As the process for forming a smooth coating film of a hydrophilic macromolecule having a uniform thickness on the surface of a guidewire using a relatively simple apparatus, a process in which a guidewire is hung perpendicularly by holding an end potion of the guidewire, a moving cell filled with a solution containing a polyisocyanate compound and a moving cell filled with a solution containing polyethylene oxide and a dicycloamidine compound are moved downward in a manner such that the guidewire passes through the cells, and a coating film of polyethylene oxide is formed on the surface of the guidewire having the coating film of polyurethane, is proposed (Patent Reference 2). As the medical appliance for insertion into the human body which can exhibit the lubricating property on the surface easily and simply and is effective for improving operability and decreasing pain of patients, a medical appliance for insertion into the human body in which the surface of the substrate is coated with a maleic acid half ester partially crosslinked with a diisocyanate, is disclosed (Patent Reference 3). However, the above processes and appliance have problems in that the process is not simple since at least two steps of forming the intermediate layer and forming the hydrophilic coating film on the surface are necessary, and that the diameter of the guidewire is increased although a small diameter is essentially preferred. In the case of using an isocyanate compound, there is the possibility that the isocyanate compound exhibits toxicity against living organs when the unreacted isocyanate compound is left remaining.

As the process for producing a guidewire for the medical use which exhibits the lubricating property at the surface in the wet condition, excellent durability in friction and a sustained lubricating property, a process in which the surface of an inner core is coated with a solution containing a mixture of a hydrophilic macromolecular substance, a macromolecular substance having carboxyl group and a compound having hydroxyl group, amino group or isocyanate group and then treated by heating, is proposed (Patent Reference 4). As the medical appliance having a coating film exhibiting excellent lubricating property, flexibility, antithrombotic property and electric insulation, a medical appliance obtained by coating with a lubricant coating film containing a hydrophilic macromolecule, a thickener and a water holding substance, followed by drying by heating, is proposed (Patent Reference 5). However, the treatment by heating causes an increase in the number of the step of production, and an apparatus such as an oven for the heating treatment is required.

As the process for producing a lubricant medical appliance which has a coating film of vapor deposition with polymerization exhibiting excellent adhesion with the surface of a substrate and having a small thickness and a coating film of a hydrophilic macromolecule which is not easily cleaved, removed or separated due to the chemical bonding to the above coating film formed by vapor deposition with polymerization and exhibits excellent lubricating property, a process in which a coating film of vapor deposition with polymerization having active hydrogen such as a coating film of a polyimide is formed on the surface of a substrate in accordance with the vapor deposition with polymerization, the formed coating film of vapor deposition with polymerization is coated with an isocyanate compound reactive with active hydrogen and a water-soluble macromolecule having active hydrogen and, then, the reaction is allowed to proceed, is proposed (Patent Reference 6). In accordance with this process, an expensive and complicated apparatus is required for forming the coating film of the vapor deposition with polymerization, the application of the process is limited to metal articles since the treatment is conducted at high temperatures, and it is necessary that the medical appliance be assembled by adding members which are not metals after the coating film of vapor deposition with polymerization has been formed although the thickness of the coating film is small.
[Patent Reference 1] Japanese Patent Application Laid-Open No. Heisei 5(1993)-184666
[Patent Reference 2] Japanese Patent Application Laid-Open No. 2004-49722
[Patent Reference 3] Japanese Patent Application Laid-Open No. Heisei 3(1991)-236854
[Patent Reference 4] Japanese Patent Application Laid-Open No. Heisei 9(1997)-154951
[Patent Reference 5] Japanese Patent Application Laid-Open No. 2004-215710
[Patent Reference 5] Japanese Patent Application Laid-Open No. 2002-95735

### DISCLOSURE OF THE INVENTION

The present invention has an object of providing a medical appliance having a metal surface which exhibits excellent lubricating property by tightly coating the metal surface with a thin layer of the hydrophilic organic compound, provides excellent operability in insertion into blood vessels and has small possibility of causing damages on inner layers of blood vessels and a process for producing the medical appliance which can produce the appliance in simple steps.

As the result of intensive studies by the present inventors to achieve the above object, it was found that a hydrophilic organic compound having a polar group could be directly fixed to the metal surface in accordance with an electrochemical reaction without intermediate layers by dipping a material for a medical appliance having a metal surface and an electrode into a solution containing a hydrophilic organic compound having a polar group and fixing the hydrophilic organic compound to the metal surface of the material for a medical appliance in accordance with an electrochemical reaction by applying a voltage between a cathode and an anode using the material for a medical appliance as the cathode and the electrode as the anode. The present invention has been completed based on the knowledge.

The present invention provides:
(1) A medical appliance having a metal surface in which a hydrophilic organic compound having a polar group is directly fixed to the metal surface in accordance with an electrochemical reaction without intermediate layers;
(2) The medical appliance described in (1), wherein the hydrophilic organic compound is a compound having a polar group having nitrogen atom or phosphorus atom;
(3) The medical appliance described in (2), wherein the polar group having nitrogen atom is amino group, imino group, amido group or imido group;
(4) The medical appliance described in (1), (2) or (3), which is an appliance inserted into a human body; and
(5) A process for producing a medical appliance which comprises dipping a material for a medical appliance having a metal surface and an electrode into a solution comprising a hydrophilic organic compound having a polar group, and fixing the hydrophilic organic compound to the metal surface of the material for a medical appliance in accordance with an electrochemical reaction by applying a voltage between a cathode and an anode using the material for a medical appliance as the cathode and the electrode as the anode.

### BRIEF DESCRIPTION OF THE DRAWINGS

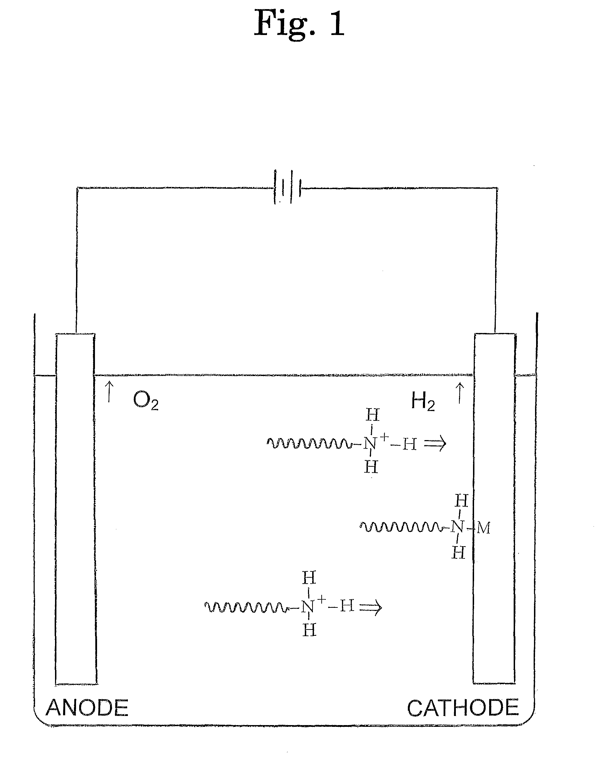

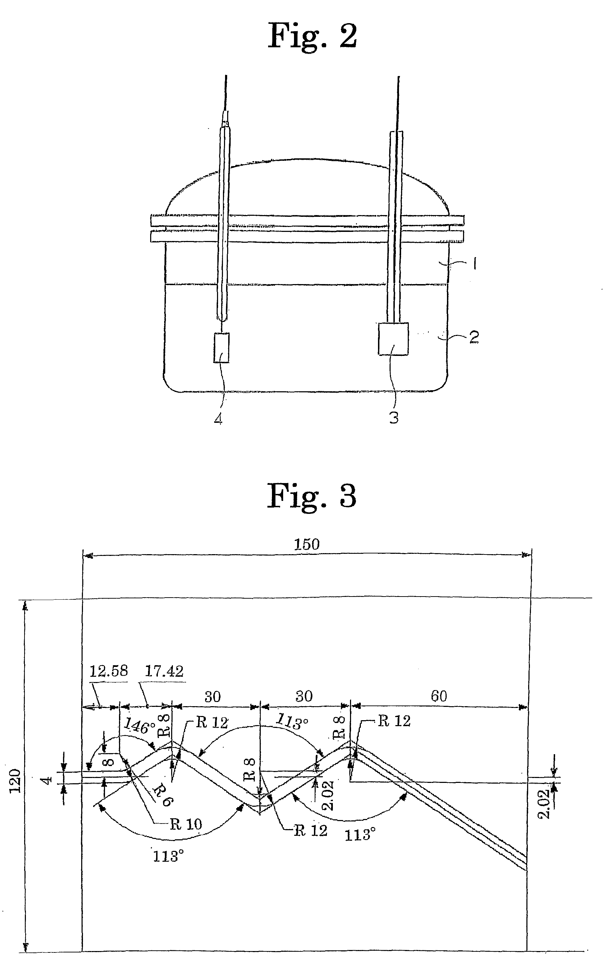

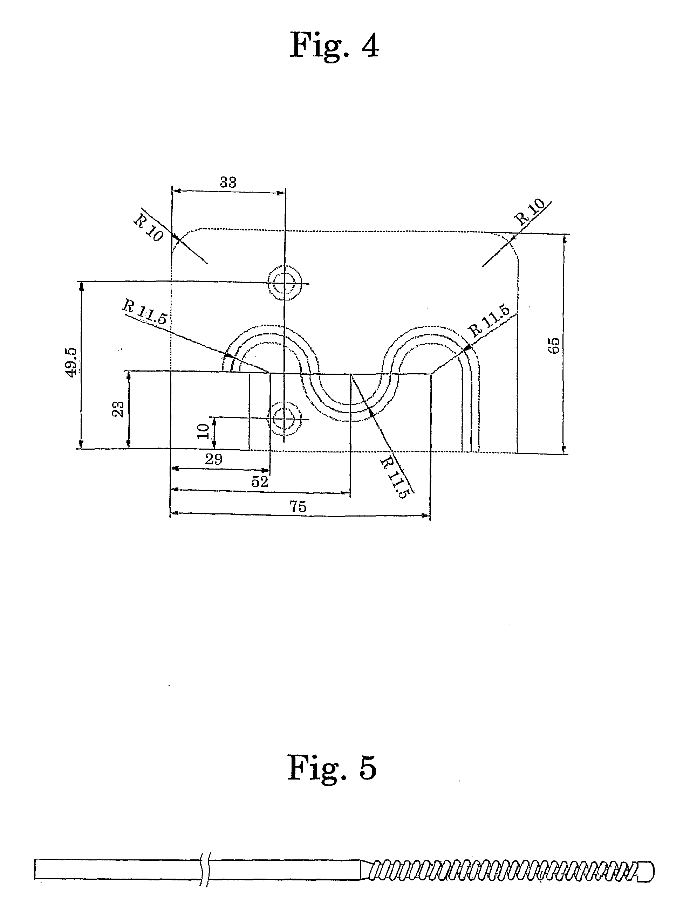

Figure 1 shows a diagram describing the electrochemical reaction used in the present invention. Figure 2 shows a diagram describing an embodiment of the process of the present invention. Figure 3 shows a diagram exhibiting a model of the body cavity used in Examples. Figure 4 shows a diagram exhibiting a model of the coronary artery used in Examples. Figure 5 shows a side view of a core wire of a guidewire used in Examples. In the Figures, reference numerals mean as follows: 1: an electrolytic cell, 2: an aqueous solution containing a hydrophilic organic compound, 3: a material for a medical appliance used as a cathode, and 4: an anode.

### THE MOST PREFERRED EMBODIMENT TO CARRY OUT THE INVENTION

The medical appliance of the present invention is a medical appliance having a metal surface in which a hydrophilic organic compound having a polar group is directly fixed to the metal surface in accordance with an electrochemical reaction without intermediate layers. The medical appliance of the present invention is inserted into portions of the human body such as blood vessels, the cardiac cavity, the gullet, the stomach cavity and intestines and advantageously used as the medical appliance for which the lubricating property of the surface is required.

Examples of the hydrophilic organic compound used in the present invention include polyethylene glycol, polyacrylamide, polyvinylpyrrolidone, polyvinyl alcohol, polyurethane, polyacrylic acid, salts of polyacrylic acid, polyethyleneimine, carboxymethylcellulose, methylcellulose, alginic acid, proteins and polysaccharides. It is preferable that the hydrophilic organic compound used in the present invention has a number-average molecular weight of 200 to 1,000,000 and more preferably 1,000 to 500,00. When the number-average molecular weight of the hydrophilic organic compound is smaller than 200, there is the possibility that the lubricating property of the surface sufficient for the medical appliance is not exhibited. When the number-average molecular weight of the hydrophilic organic compound exceeds 1,000,000, the compound is not easily treated in the electrochemical reaction, and there is the possibility that blood corpuscles and the like are taken into the hydrophilic coating film at the surface of the medical appliance to cause problems.

In the present invention, the polar group in the hydrophilic organic compound is a group having an atom having an electronegativity different from that of carbon atom. Examples of the polar group include amino group (-NH₂), imino group (=NH), amido group (-CONH₂), imido group (-CONHCO-), epoxy group, isocyanate group (-NCO), cyano group (-CN), nitro group (-NO₂), mercapto group (-SH), thio group (-S-) and phosphino group (-PH₂). The hydrophilic organic compound may have a single group or a combination of two or more groups among the above groups. As for a selected group, a single group or a plurality of the same group may be present. Hydrophilic organic compounds having a polar group having nitrogen atom or phosphorus atom among the above polar groups are preferable. Hydrophilic organic compounds having amino group, imino group, amido group or imido group are more preferable.

Examples of the hydrophilic organic compound having a polar group used in the present invention include polyethylene glycol having amino groups at both ends and polyethylene glycol having epoxy groups at both ends. Polyethylene glycol having amino group at both ends can be produced, for example, by forming polyethylene glycol diallyl ether by the reaction of polyethylene glycol and allyl chloride, followed by adding ammonia to the double bond. For preparation of polyethylene glycol having epoxy group at both ends, polyethylene glycol having glycidyl group at both ends can be produced, for example, by forming polyethylene glycol having 2-hydroxy-3-chloropropyl group at both ends by the reaction of polyethylene glycol and epichlorohydrin, followed by the ring closure with dehydrochlorination.

In the production of the medical appliance of the present invention, the electrochemical reaction for fixing the hydrophilic organic compound having a polar group to the surface of the metal is the reaction in which the electrochemical potential is varied depending on other factors in the electrochemical system and goes through steps such as movement of substances toward the surface of the electrode, adsorption of the substances at the surface of the electrode, dissociation of the substances at the surface of the electrode and donation and receipt of electrons. Figure 1 shows a diagram describing the electrochemical reaction used in the present invention. A proton is added to amino group at the end of the polyethylene glycol chain to form a quaternary ammonium group, which moves to the cathodes. The quaternary ammonium group is adsorbed at the surface of the cathode and dissociated into amino group and proton at the surface of the cathode. An electron is provided to the proton at the cathode, and hydrogen gas is formed. Since electrons of the lone pair in the amino group is shared with free electrons in the metal of the cathode, a strong bond is formed between the amino group at the end of the polyethylene glycol chain and the metal of the cathode, and the strong bond is maintained after the electric current is stopped.

In the medical appliance of the present invention, it is preferable that the thickness of the coating film formed with the hydrophilic organic compound is 0.01 to 20 µm and more preferably 0.1 to 10 µm. When the thickness of the coating film is smaller than 0.01 µm, there is the possibility that the lubricating property of the surface sufficient for the medical appliance is not exhibited. When the thickness exceeds 20 µm, the lubricating property is not improved even when the thickness is increased, and there is the possibility that blood corpuscles are taken into the coating film to cause problems.

The medical appliance of the present invention can be advantageously used as the appliance inserted into the human body. Examples of the medical appliance inserted into the human body include appliances inserted for a long period of time such as stents, coils for emboli, artificial cardiac valves, pace makers and artificial blood vessels and appliances inserted for a limited period of time such as guidewires, catheters and filters for removing thrombi.

The process for producing a medical appliance of the present invention comprises dipping a material for a medical appliance having a metal surface and an electrode into a solution comprising a hydrophilic organic compound having a polar group, and fixing the hydrophilic organic compound to the metal surface of the material for a medical appliance in accordance with an electrochemical reaction by applying a voltage between a cathode and an anode using the material for a medical appliance as the cathode and the electrode as the anode.

Figure 2 shows a diagram describing an embodiment of the process of the present invention. Into an aqueous solution 2 comprising a hydrophilic organic compound having a polar group which is stored in an electrolytic cell 1, a material for a medical appliance 3 used as the cathode and an anode are dipped. It is preferable that the concentration of the hydrophilic organic compound is 1 to 30% by weight and more preferably 5 to 15% by weight. When the concentration of the hydrophilic organic compound is smaller than 1% by weight, the thickness of the coating film is small, and there is the possibility that the sufficient lubricating property is not exhibited. When the concentration of the hydrophilic organic compound exceeds 30% by weight, there is the possibility that blood corpuscles are taken into the coating film to cause problems.

In the process of the present invention, it is preferable that an inorganic electrolyte is dissolved into the aqueous solution comprising the hydrophilic organic compound in advance. Examples of the inorganic electrolyte dissolved into the above aqueous solution include sodium chloride and potassium chloride. By dissolving the inorganic electrolyte into the above aqueous solution in advance, the aqueous solution is made electrically conductive, and electrons can move between the anode and the cathode. It is preferable that the concentration of the inorganic electrolyte is 1 to 5% by weight and more preferably 2 to 4% by weight. When the concentration of the inorganic electrolyte is smaller than 1% by weight, there is the possibility that the electric conductivity of the aqueous solution decreases. When the concentration of the inorganic electrolyte exceeds 5% by weight, there is the possibility that ions in the inorganic electrolyte are adsorbed at the surface of the metal.

In the process of the present invention, it is preferable that the voltage applied between the anode and the cathode is 0.1 to 10 V and more preferably 2 to 7 V. When the voltage is smaller than 0.1 V, there is the possibility that it takes a long time for forming the coating film by fixing the hydrophilic organic compound to the surface of the cathode. When the voltage exceeds 10 V, there is the possibility that bubbles are formed in a great amount at the surface of the metal, and a uniform coating film is not formed.

In the process of the present invention, it is preferable that the current density per the surface area of the cathode is 1×10⁻⁷ to 5×10⁻⁵ A/dm² and more preferably 5×10⁻⁶ to 1×10⁻⁵ A/dm². When the current density is smaller than 1×10⁻⁷ A/dm², there is the possibility that it takes a long time for forming the coating film by fixing the hydrophilic organic compound to the surface of the cathode. When the current density exceeds 5×10⁻⁵ A/dm², there is the possibility that bubbles are formed in a great amount at the surface of the metal, and a uniform coating film is not formed.

The electrochemical reaction in the process for producing a medical appliance of the present invention can be conducted in an aqueous solution at the room temperature. Therefore, even when members other than metals having poor heat resistance or solvent resistance are used in the medical appliance, the electrochemical reaction can be conducted using an intermediate product having such members. Since the coating with the hydrophilic organic compound is conducted on the metal surface having the electric conductivity alone, there is no possibility that the coating with a film takes place at unnecessary portions unlike the coating with a coating material.

In the process in which the medical appliance having a metal surface is dipped into the solution containing the hydrophilic organic compound having a polar group and the hydrophilic organic compound is fixed to the surface of the metal in accordance with the electrochemical reaction, a coating film of the hydrophilic organic compound having a uniform thickness can be formed at the end of the process even when the amount of the attached hydrophilic organic compound is uneven in various portions in the initial stage since the current density is smaller at portions having a greater amount of the attached hydrophilic organic compound and greater at portions having a smaller amount of the attached hydrophilic organic compound, and the hydrophilic organic compound is attached selectively at portions having a smaller amount of the attached hydrophilic organic compound.

In the process of the present invention, the thickness of the hydrophilic organic compound can be controlled by the molecular weight of the hydrophilic organic compound. For example, when polyethylene glycol having amino group at both ends is used, the thickness of the coating film is about one half of the chain length of polyethylene glycol since amino groups at both ends are bonded to the surface of the metal. Therefore, a polyethylene glycol having a greater molecular weight is used when a coating film having a greater thickness is necessary, and a polyethylene glycol having a smaller molecular weight is used when a coating film having a smaller thickness is necessary. In this manner, the coating film of the hydrophilic organic compound having the selected thickness can be formed.

### EXAMPLES

The present invention will be described more specifically with reference to examples in the following. However, the present invention is not limited to the examples.

In the Example and the Comparative Example, the evaluation of a guidewire was conducted in accordance with the following methods.

### (1) Property for insertion

A model of the body cavity made of polytetrafluoroethylene and having a long cavity having a length of 900 mm and an inner diameter of 2 mm as shown in Figure 3 was dipped into a thermostatted water tank kept at 37°C. A guidewire was inserted into the model at a speed of 200 mm/min, and the load, i.e., the resistance to pushing-in, was measured using a load cell.

### (2) Durability

The durability was evaluated by the resistance to pushing-in after the test of the property for insertion was repeated 100 times. After the test of the property for insertion was repeated 100 times, the sample was taken out. The coil portion of the sample was observed by a stereomicroscope, and the condition of attachment of the hydrophilic organic compound was observed.

### (3) Property for transfer of torque

A model of the human coronary artery made of polytetrafluoroethylene shown in Figure 4 was dipped into a thermostatted water tank kept at 37°C. A guidewire was inserted into the model. The near end portion of the guidewire for the handling was rotated by 720 degrees in the clockwise direction while the guidewire was kept at a bent condition, and the condition of rotation of the tip portion was observed.

### Example 1

A guidewire was prepared using a wire rod of stainless steel SUS 316 having a shape having a length of 1,400 mm and an outer diameter of 340 µm and a coil portion at the far end portion as shown in Figure 5.

A solution obtained by dissolving 12% by weight of polyethylene glycol having aminopropyl group at both ends (the number-average molecular weight: 1,000) and 3.0% by weight of sodium chloride into deionized water in an amount of 2.0 liters was placed into a glass electrolytic cell having an inner diameter of 135 mm at the bottom face and a height of 200 mm.

After the core wire was defatted, the core wire was wound into a coil shape having a diameter of 45 mm and a pitch of 10 mm and placed into the electrolytic cell in a manner such that the central axis of the coil was placed at the central axis of the electrolytic cell and the distance from the liquid surface to the coil and the distance from the bottom surface to the coil are the same. A platinum electrode having a length of 150 mm was placed at the position of the central axis of the coil in a manner such that the lower end portion of the electrode protruded below the lower end portion of the coil by about 10 mm.

Using the platinum electrode as the anode and the core wire wound into the coil shape as the cathode, the electrochemical reaction was conducted by passing electric current for 3 minutes under application of a voltage of 5.0 V between the electrodes while the liquid was stirred by the rotation of a stirrer rod using a magnetic stirrer. After the electric current was stopped, the core wire wound into a coil shape was taken out and washed with deionized water, and a guidewire having a coating film of polyethylene glycol formed on the surface was obtained. The thickness of the coating film of polyethylene glycol was 1.2 µm.

In the test of the property for insertion, the resistance to pushing-in was 0.26 N. The resistance to pushing-in was 0.28 N after the test of the property for insertion was repeated 100 times, and no cleavage of the coating film was found. The property for transfer of torque was excellent.

### Comparative Example 1

In a manner similar to that in Example 1, a core wire was wound into a coil shape, dipped into a solution prepared by dissolving 12% by weight of polyethylene glycol having aminopropyl group at both ends (the number-average molecular weight: 1,000) and 3.0% by weight of sodium chloride into deionized water for 3 minutes, and the liquid was stirred by rotating a stirrer rod using a magnetic stirrer without passing electric current.

When the core wire was taken out of the solution and washed with deionized water, no coating film was formed on the core wire.

In the test of the property for insertion, the resistance to pushing-in was 2.53 N. The resistance to pushing-in was 2.53 N after the test of the property for insertion was repeated 100 times. The property for transfer of torque was poor.

The results of Example 1 and Comparative Example 1 are shown in Table 1.

**Table 1**

| | Resistance to pushing-in | Resistance to pushing-in after 100 insertions | | Property for transfer of torque |
|---|---|---|---|---|
| | (N) | resistance (N) | cleavage of coating film | |
| Example 1 | 0.26 | 0.28 | none | excellent |
| Comparative Example 1 | 2.53 | 2.53 | - | poor |

### INDUSTRIAL APPLICABILITY

The medical appliance of the present invention exhibits the excellent lubricating property of the surface, has strong bond between the coating film containing the hydrophilic organic compound and the metal surface since the hydrophilic organic compound is directly bonded to the metal surface in accordance with the electrochemical reaction without intermediate layers, causes no cleavage or removal of the coating film during the use of the medical appliance and can prevent elution of the metal components. The thickness of the coating film on the metal surface can be decreased since no intermediate layers are present. The medical appliance of the present invention can be advantageously used as the appliance inserted into the human body such as guidewires and stents. In accordance with the process of the present invention, the medical appliance in which the hydrophilic organic compound is directly fixed to the metal surface without intermediate layers can be produced efficiently in a short time under a mild condition.

## Claims

1. A medical appliance having a metal surface in which a hydrophilic organic compound having a polar group is directly fixed to the metal surface in accordance with an electrochemical reaction without intermediate layers.

2. The medical appliance according to Claim 1, wherein the hydrophilic organic compound is a compound having a polar group having nitrogen atom or phosphorus atom.

3. The medical appliance according to Claim 2, wherein the polar group having nitrogen atom is amino group, imino group, amido group or imido group.

4. The medical appliance according to Claim 1, Claim 2 or Claim 3, which is an appliance inserted into a human body.

5. A process for producing a medical appliance which comprises dipping a material for a medical appliance having a metal surface and an electrode into a solution comprising a hydrophilic organic compound having a polar group, and fixing the hydrophilic organic compound to the metal surface of the material for a medical appliance in accordance with an electrochemical reaction by applying a voltage between a cathode and an anode using the material for a medical appliance as the cathode and the electrode as the anode.
